# EUROPEAN PATENT SPECIFICATION

(11) **EP 3 621 672 B1**
(45) Date of publication and mention of the grant of the patent: **30.06.2021**
(21) Application number: 17725820.9
(22) Date of filing: 12.05.2017
(51) Int. Cl.: A61M 1/16

(54) **INFUSATE SLEEVE**
HÜLSE FÜR INFUSIONSLÖSUNG
MANCHON DE SOLUTION INTRAVEINEUSE

(43) Date of publication of application: 18.03.2020
(73) Proprietor: Medtronic, Inc., Minneapolis, MN 55432 (US)
(72) Inventor: LURA, David B., Maple Grove, Minnesota 55311 (US); KOREC, Bartosz, Palm Harbor, Florida 34683 (US); XUE, Yueqiang, Shanghai 200126 (CN); HUANG, Jin, Shanghai 201114 (CN)
(74) Representative: Maschio, Antonio
(86) International application number: PCT/US2017/032354
(87) International publication number: WO 2018/208313

(56) References cited:
- EP-A1- 0 714 668
- WO-A1-2013/077844
- WO-A1-2017/004449
- US-A1- 2017 021 076

## Description

### FIELD OF THE INVENTION

The invention relates to infusate sleeves for use in dialysis and related systems and methods. The infusate sleeves include a sleeve body, a filter inside of the sleeve body, a rigid disk covering an opening in the top of the sleeve body, a draw tube connected to the rigid disk and extending downwardly into the sleeve body, and a fluid connector fluidly connected to the draw tube for connection to a dialysis system.

### BACKGROUND

During priming of a dialysis system and during dialysis treatment, specific concentrations of specific solutions, such as sodium chloride, sodium bicarbonate, and cation infusates, must be added to the dialysate flow path. Further, many cations, such as potassium, calcium and magnesium, can cross the dialyzer and be removed from a patient during dialysis. The cations must be added back into the dialysate to maintain the concentration of the cations at a desired level. Sodium bicarbonate can be used during dialysis as a buffer to control the pH of the dialysate and to treat acidosis by delivering bicarbonate across the dialysis membrane to the patient receiving a treatment. Because the relative concentrations of the sodium chloride, sodium bicarbonate, and cations can vary from patient to patient or when used for either priming or treatment, each of the solutions must be added from separate containers. Before each use, the separate containers must be cleaned and sterilized driving up costs and time.

There is a need for systems and methods that can use inexpensive disposable components within reusable containers for holding each of the substances to be added to a dialysate flow path during either treatment or priming. There is a need for the disposable components that allow solid infusate sources to be dissolved, creating infusate solutions of known concentration while preventing any particulate matter from entering the dialysis system.

WO2017004449 discloses a sleeve of a collection canister, wherein a draw tube extends from a cap to a bottom portion of the sleeve and is provided with a filter element.

### SUMMARY OF THE INVENTION

The first aspect of the invention relates to an infusate sleeve according to claim 1. The infusate sleeve includes a sleeve body; a filter inside of the sleeve body and sealed to the sleeve body separating the sleeve body into a top portion and a bottom portion; a rigid disk sealed to a top portion of the sleeve body; the rigid disk covering an opening in the top portion of the sleeve body; a second opening in the bottom portion of the sleeve body; a draw tube connected to the rigid disk; the draw tube downwardly extending from the rigid disk through the filter and into the bottom portion; and a fluid connector connected to an outside of the rigid disk; the fluid connector in fluid connection with the draw tube.

In any embodiment, the filter and rigid disk can be heat sealed to the sleeve body.

In any embodiment, the sleeve body can be flexible.

In any embodiment, the sleeve body can be rigid.

In any embodiment, the fluid connector can be a bi-channel connector.

In any embodiment, a first channel in the bi-channel connector can be fluidly connected to the draw tube.

In any embodiment, the infusate sleeve can include a cup covering the opening in the bottom portion of the sleeve body; the cup sealed to the sleeve body.

In any embodiment, the filter can be sealed to the cup.

The second aspect of the invention is drawn to an infusate container according to claim 10. The infusate container includes an infusate container body; the infusate sleeve of the first aspect of the invention inside the container body; and a cap; wherein the fluid connector extends through an opening in the cap.

In any embodiment, the infusate sleeve can be flexible.

In any embodiment, the infusate sleeve can be rigid.

The third aspect of the invention is drawn to a dialysis system according to claim 12. The dialysis system includes a dialysate flow path; one or more fluid connectors fluidly connecting one or more infusate containers of the second aspect of the invention to the dialysate flow path; and at least one pump connected to a fluid line fluidly connected to the fluid connectors.

In any embodiment, the fluid connector can be a bi-channel connector.

In any embodiment, a first channel of the bi-channel connector can fluidly connect the draw tube to a first fluid line; and a second channel of the bi-channel connector can fluidly connect the infusate sleeve to a second fluid line.

In any embodiment, the infusate sleeve can contain sodium chloride, sodium bicarbonate, a cation infusate, or combinations thereof.

The fourth aspect of the disclosure is drawn to a method, which is not part of the claimed invention. In any embodiment, the method can include the steps of flowing water into an infusate container the second aspect of the invention, wherein the infusate container contains a solid infusate; dissolving at least a portion of the solid infusate to make an infusate solution; and flowing the infusate solution into a dialysate flow path.

In any embodiment, the step of flowing water into the infusate container can include flowing water through a first channel of a bi-channel connector; and the step of flowing the infusate solution into the dialysate flow path can include flowing the infusate solution through a second channel of the bi-channel connector.

In any embodiment, the step of dissolving at least a portion of the solid infusate to make an infusate solution can include making a saturated infusate solution.

In any embodiment, the infusate can be sodium bicarbonate, sodium chloride, a cation infusate, or a combination thereof.

### BRIEF DESCRIPTION OF THE DRAWINGS

FIG.1 shows a flexible infusate sleeve with for use in dialysis.
FIG. 2 shows a flexible infusate sleeve inside of an infusate container.
FIG. 3 shows a rigid infusate sleeve for use in dialysis.
FIG. 4 shows a rigid infusate sleeve inside of an infusate container.
FIG.'s 5A-D show bi-channel connectors for use with the infusate sleeve.
FIG.'s 6A-C show an infusate holder for use with integrally formed disposable infusate sleeves.
FIG.'s 7A-B show an infusate holder for use with separate disposable infusate sleeves.

### DETAILED DESCRIPTION OF THE INVENTION

Unless defined otherwise, all technical and scientific terms used herein generally have the same meaning as commonly understood by one of ordinary skill in the relevant art.

The articles "a" and "an" are used to refer to one or to over one (*i.e.,* to at least one) of the grammatical object of the article. For example, "an element" means one element or over one element.

The term "bi-channel connector" refers to a fluid connector having two channels for fluid movement in either direction.

The term "bottom portion" of a container refers to the portion of the container or component near or at a lowest elevation.

The term "cap" refers to a portion of a component covering an opening.

The term "cation infusate" refers to cations that are added to a dialysate during dialysis therapy.

The term "channel" refers to any pathway within a component through which a fluid may travel.

The term "comprising" includes, but is not limited to, whatever follows the word "comprising." Use of the term indicates the listed elements are required or mandatory but that other elements are optional and may be present.

The terms "connect" or "connected" refer to a physical contact that resists movement between two or more components. The connections can be detachable and reattached between the two or more components.

The term "consisting of' includes and is limited to whatever follows the phrase "consisting of." The phrase indicates the limited elements are required or mandatory and that no other elements may be present.

The term "consisting essentially of' includes whatever follows the term "consisting essentially of' and additional elements, structures, acts or features that do not affect the basic operation of the apparatus, structure or method described.

The term "cup" refers to a component having a base and upwardly extending sidewalls defining an interior space.

A "dialysate flow path" is a route in which a fluid can travel during dialysis.

"Dialysis" or "dialysis therapy" is a type of filtration, or a process of selective diffusion through a membrane. Dialysis removes solutes of a specific range of molecular weights via diffusion through a membrane from a fluid to be dialyzed into a dialysate. During dialysis, a fluid to be dialyzed is passed over a filter membrane, while dialysate is passed over the other side of that membrane. Dissolved solutes are transported across the filter membrane by diffusion between the fluids. The dialysate is used to remove solutes from the fluid to be dialyzed. The dialysate can also provide enrichment to the other fluid.

A "dialysis system" is a system comprising a dialyzer, pumps, valves and fluid lines that is used to carry out a dialysis session.

A "disk" is a planar component having a substantially round or circular shape.

The terms "dissolving" or to "dissolve" refer to causing a solid or gas to become incorporated into a liquid to form a solution.

"Downwardly extending" or to "extend downwardly" refers to a component positioned from a higher elevation to a lower elevation

A "draw tube" is a fluid connector extending into an interior space of a component.

A "filter" is a component that inhibits the passage particulate matter conveyed by a fluid or solution while allowing the passage of the fluid or solution.

The term "flexible" refers to a component having a shape that can be changed or bent.

The terms "flowing" or to "flow" "refer to the movement of a fluid, gas, or mixtures thereof.

A "fluid" is a liquid substance optionally having a combination of gas and liquid phases in the fluid. Notably, a liquid can therefore also have a mixture of gas and liquid phases of matter.

The term "fluid connector,""fluidly connectable," or "fluidly connected" refers to the ability to pass fluid, gas, or mixtures thereof from one point to another point. The two points can be within or between any one or more of compartments, modules, systems, and components, all of any type.

The term "fluid line" refers to a fluid pathway.

The term "fluid pump" or "pump" refers to any device that causes the movement of fluids or gases by applying suction or pressure.

The term "frit filter" refers to porous glass made by sintering together glass particles into a porous body.

The term "heat sealing" or "heat sealed" refers to the use of heat to unite or connect two thermoplastic materials.

An "infusate container" is a container adapted to contain one or more fluids for use in dialysis. The infusate container can at times hold dry chemicals that are later able to be reconstituted with a fluid to form a further useable fluid within the system.

The term "infusate container body" refers to the outer boundaries of a container enclosing the interior of the infusate container.

An "infusate sleeve" is a disposable component insertable into an infusate container into which the infusate is placed.

An "infusate solution" is any substance or substances dissolved in water or dialysate to be added to a dialysate flow path.

The term "mesh" refers to a component made of strands of fibers with spaces between the fibers to allow fluid or gas to flow through the mesh.

An "opening" is a portion of a component having a defined void space.

The term "outside" refers to the portion of a component on the exterior of the component.

The term "rigid" refers to a component having a substantially stiff structure that resist bending and is not generally flexible.

The term "saturated" refers to the highest amount of a substance that can be dissolved in a solvent at a given temperature.

The term "sealed" refers to a connection between two components through which a fluid, gas, and mixtures thereof cannot pass.

The term "sleeve body" refers to the outer boundaries of a container enclosing an interior of a container.

The term "solid infusate" refers to any substance intended to be added to a dialysate flow path in the solid form of matter.

The term "top portion" of a container refers to the portion of the container or component near or at a highest elevation.

### Infusate Sleeve

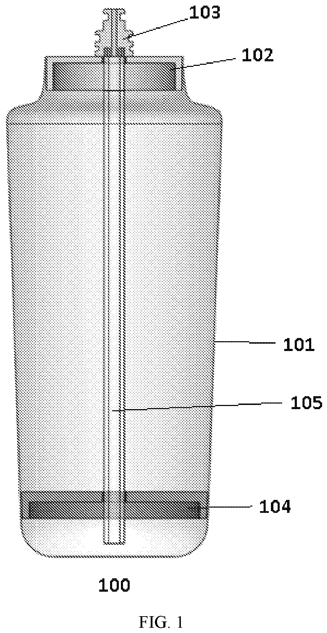

FIG. 1 illustrates a non-limiting embodiment of an infusate sleeve **100.** The infusate sleeve **100** can be a flexible material into which infusates for use with a dialysis system can be placed. The infusate sleeve **100** has a sleeve body **101** with an opening in the top portion of the sleeve body **101.** A rigid disk **102** can be sealed to the top portion of the sleeve body **101,** covering the opening. The rigid disk **102** can have a fluid connector **103** connected to the outside of the rigid disk **102** for fluidly connecting to one or more fluid lines in a dialysis system. The fluid connector **103** can connect to a draw tube **105** through which an infusate solution can be drawn out of the infusate sleeve **100** and into the dialysis system. A pump connected to the fluid line can provide the driving force for the movement of fluid into and out of the infusate sleeve **100** through fluid connector **103.**

The rigid disk **102** provides a stable top for the infusate sleeve **100,** allowing easier connection of the fluid connector **103** to a dialysis system. The rigid disk **102** can be sealed to the sleeve body **101** of the infusate sleeve **100** by any means known in the art that can create a seal, including heat sealing, molding, gluing, soldering, and mechanical fixation. The infusate sleeve **100** also includes a filter **104** sealed to the sleeve body **101.** The filter **104** separates the infusate sleeve **100** into a top portion and a bottom portion. A solid infusate can be placed on top of the filter **104.** Water can be into the infusate sleeve **100** to dissolve the infusate with the resulting solution flowing below the filter **104** into the bottom portion. The solids cannot pass through the filter **104,** and remain in the top portion. The filter **104** can be any type of filter known in the art capable of preventing solid or particulate matter from passing through the filter **104,** including a frit filter or a mesh filter. The filter **104** can be sealed to the sleeve body **101** by any method known in the art capable of creating a seal between the sleeve body **101** and the filter **104,** including heat sealing. The draw tube **105** can extend downwardly from the rigid disk **102** through the filter **104.** The solution in the bottom portion of the infusate sleeve **100** can be drawn through the draw tube **105** and added to a dialysate flow path with a pump positioned on a fluid line connected to the draw tube **105.**

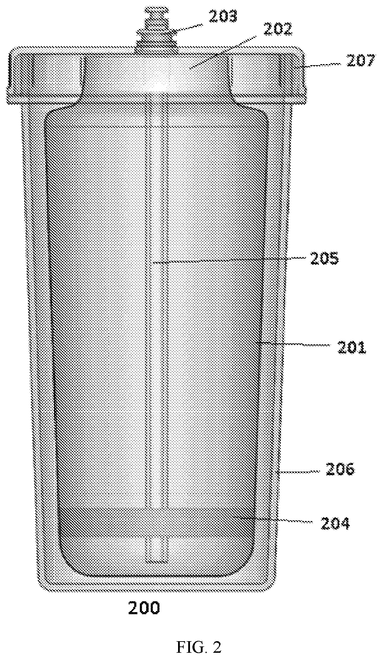

For use with a dialysis system, the infusate sleeve **201** can be inserted into an infusate container **20** as illustrated in FIG. 2. The infusate sleeve **201** can be inserted into the infusate container **200.** The infusate container **200** has a rigid infusate container body **206** that provides a stable and rigid structure, allowing the fluid connector **203** to be easily connected to and disconnected by transferring the force when connecting from the flexible infusate sleeve **201** to the dialysis system. The infusate sleeve **201** can include a rigid disk **202** with a fluid connector **203** on the outside of the rigid disk **202** connected to a draw tube **205.** A filter **204** prevents solid or particulate matter from being added to a dialysate flow path through draw tube **205.** The rigid disk **202** can engage with a cap **207** on the infusate container **200.** The cap **207** can have an opening through which the fluid connector **203** can extend for connection to the dialysis system.

The infusate sleeve **201** can be a disposable part, while the infusate container **200** is reusable. The infusate sleeve **201** thus allows a cheap method to contain and use infusates, while eliminating the need for cleaning and sterilization of the infusate container **200.** For each dialysis session, the user need only insert a new infusate sleeve **201** into the reusable infusate container **200.** After placing a solid infusate into the infusate sleeve **201** above the filter **204,** purified water from the dialysis system can be added to the infusate sleeve **201** to dissolve the solid infusate, creating an infusate solution that flows through the filter **204** into the bottom portion of the infusate sleeve **201.** The infusate sleeve **201** expands upon filling with water to accommodate the larger volume within the infusate sleeve **201.** For use with solid infusates, water can be added to the infusate sleeve **201** to dissolve the infusate, making a solution of known concentration for addition to a dialysis system. For example, an excess amount of the solid infusate can be added to the infusate sleeve **201.** Water can be added to the infusate sleeve **201** in an amount insufficient to dissolve all of the solid infusate. The resulting infusate solution will be saturated in the infusate. At known temperatures, the concentration of the infusate in the saturated infusate solution will be known. Alternatively, a specified amount of solid infusate and water can be added to the infusate sleeve **201** to generate an infusate solution of known concentration.

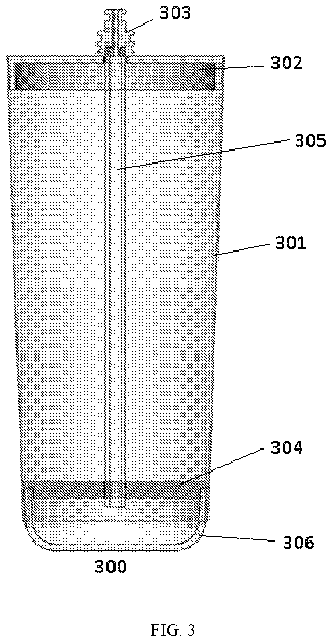

FIG. 3 illustrates an alternative infusate sleeve **300** with openings in the top and bottom of the sleeve body **301.** As with the infusate sleeve **100** illustrated in FIG. 1, the infusate sleeve **300** in FIG. 3 has an opening in the top portion of the sleeve body **301.** A rigid disk **302** can be sealed to the top portion of the sleeve body **301,** covering the opening. The rigid disk **302** can have a fluid connector **303** for connection to one or more fluid lines in a dialysis system. The fluid connector **303** can connect to a draw tube **305** through which an infusate solution can be drawn out of the infusate sleeve **300** and into the dialysis system. A pump connected to the fluid line can provide the driving force for the movement of fluid into and out of the infusate sleeve **300** through fluid connector **303.** The infusate sleeve **300** also has an opening in the bottom portion of the sleeve body **301.** A cup **306** can cover the opening in the bottom portion of the sleeve body **301** and can be sealed to the sleeve body **301** by any means known in the art. The cup **306** is a rigid component that, with the rigid disk **302,** maintains the shape of the infusate sleeve **300.** A filter **304** can be sealed to the infusate sleeve **300** and sit on top of the cup **306.** The infusate sleeve **300** can be a rigid component to further maintain the shape of the infusate sleeve **300.** By using a rigid infusate sleeve **300** pressure buildup due to addition of water into the infusate sleeve **300** can be eliminated.

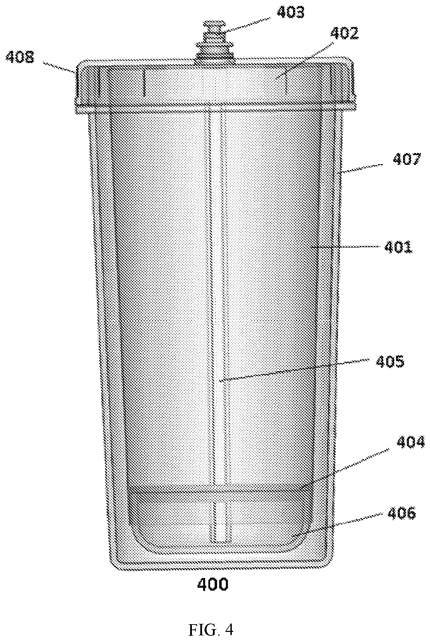

FIG. 4 illustrates an infusate sleeve **401** as illustrated in FIG. 3 inside of an infusate container **400.** The infusate sleeve **401** can be inserted into the infusate container **400.** The infusate container **400** has a rigid infusate container body **407** that provides a stable and rigid structure, allowing the fluid connector **403** to be easily connected to and disconnected by transferring the force when connecting from the infusate sleeve **401** to the dialysis system. The infusate sleeve **401** can include a rigid disk **402** with a fluid connector **403** connected to a draw tube **405.** A filter **404** prevents solid or particulate matter from being added to a dialysate flow path through draw tube **405.** The rigid disk **402** can engage with a cap **408** on the infusate container **400.** The cap **408** can have an opening through which the fluid connector **403** can extend for connection to the dialysis system. The cup **406** can sit on top of a base of the infusate container **400.** Because the rigid disk **402** and cup **406** hold the infusate sleeve **401** in shape, the infusate sleeve **401** does not need to expand when water is added to the infusate sleeve **401.** Instead, the infusate sleeve **401** fills the infusate container **400,** eliminating the buildup of pressure in the infusate sleeve **401** as fluid is added to the infusate sleeve **401.** By eliminating the pressure buildup, inadvertent popping of the infusate sleeve **401** can be avoided.

The infusate sleeves can be used with any infusate necessary for priming, disinfecting, or providing treatment with a dialysis system. The infusate sleeves can contain sodium bicarbonate, sodium chloride, cation infusates, or combinations thereof. Any number of infusate sleeves and infusate containers can be used with a dialysis system, including 1, 2, 3, 4, 5, or more. A single cation infusate sleeve can be used for all cation infusates, or separate cation infusate sleeves can be used for each cation to be added to a dialysate flow path, such as potassium, magnesium, and calcium.

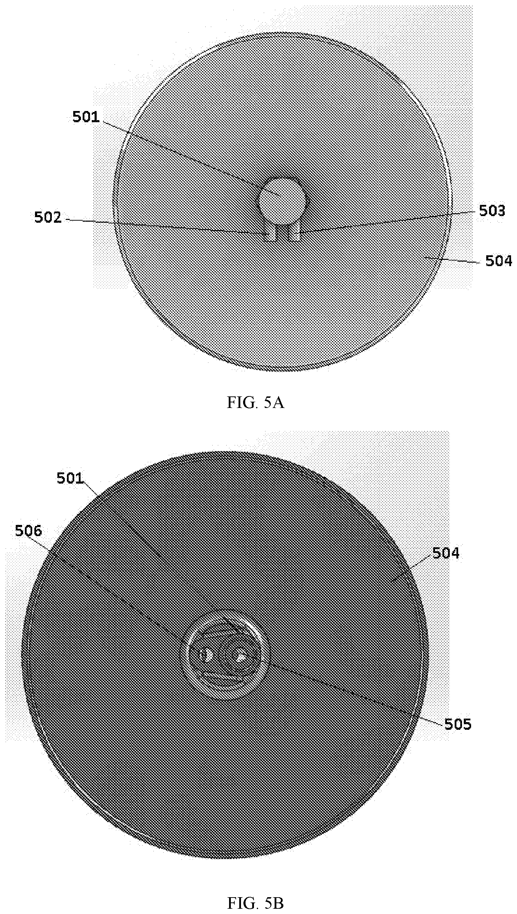

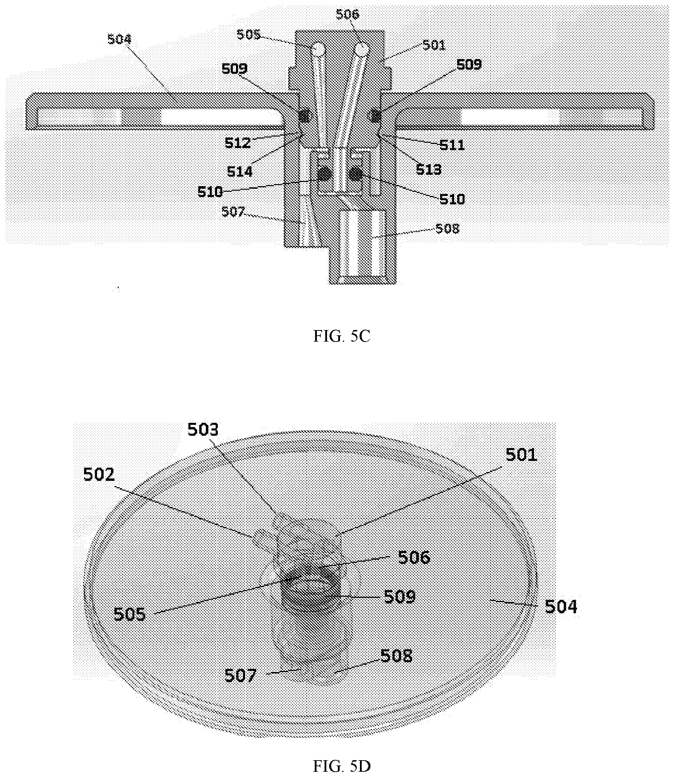

Any of the fluid connectors described can be bi-channel connectors. FIG.'s 5A-D illustrate one embodiment of a bi-channel connector **501.** FIG. 5A is a top view of a bi-channel connector **501,** FIG. 5B is a top cut-away view of the bi-channel connector **501,** FIG. 5C is a cross-sectional view of the bi-channel connector **501,** and FIG. 5D is a transparent view of the bi-channel connector **501.** Each view shows a rigid disk **504** and bi-channel connector **501.** The connector **501** includes fluid inlet **502** for moving fluid into the infusate sleeve (not shown in FIG.'s 5A-D), and fluid outlet **503** for removing fluid from the infusate sleeve. The fluid inlet **502** is connected to a first channel **505,** and the fluid outlet **503** is connected to a second channel **506,** as illustrated in FIG. 5B. The first channel **505** is connected to an infusate sleeve inlet **507** for movement of fluid into the infusate sleeve. The second channel **506** is connected to an infuse sleeve outlet **508** for movement of fluid out of the infusate sleeve. The infuse sleeve outlet **508** can be fluidly connected to a draw tube (not shown in FIG.'s 5A-D) that extends downwardly into the container body. An o-ring or other sealing member **509** can be included to prevent leakage around the rigid disk **504** of the infusate sleeve where the rigid disk **504** contacts the bi-channel connector **501.** As illustrated in FIG. 5C, a second o-ring **510** can be included to prevent leakage between the first channel **505** and second channel **506.** The rigid disk **504** can also include protrusions **511** and **512** which can engage with complementary indentations **513** and **514** on the bi-channel connector **501** to securely fasten the bi-channel connector **501** in place on the rigid disk **504** without the need to twist or screw the bi-channel connector **501**

The fluid inlet **502** and fluid outlet **503** can be fluidly connected to a dialysis system through separate fluid lines. By using separate fluid lines for influx and efflux of fluid to and from the infusate sleeve, additional water can be added to the infusate sleeve during priming or use without contamination of the infusates within the container.

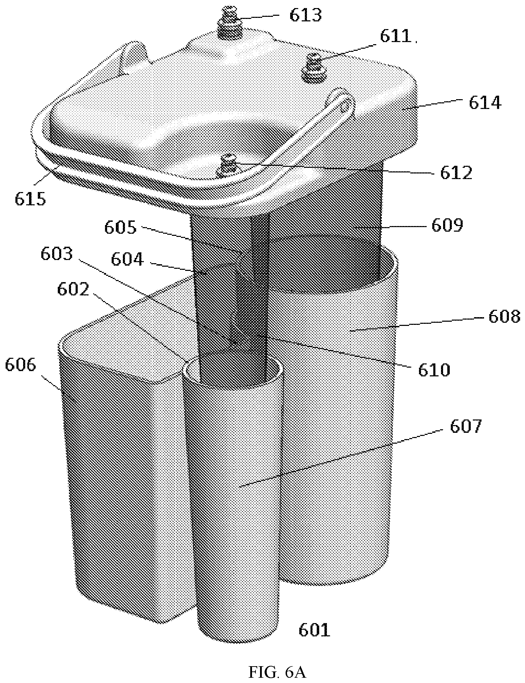

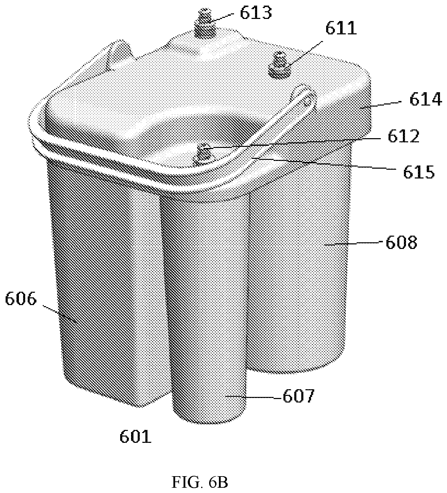

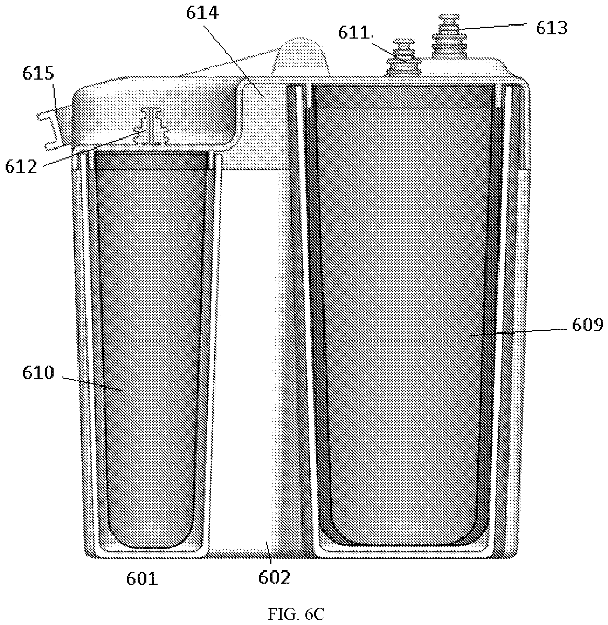

FIG.'s 6A-C illustrate an infusate holder **601** with a disposable lid **614** integrally formed with disposable sodium bicarbonate infusate sleeve **609** and sodium chloride infusate sleeve **610.** Additional disposable or reusable infusate sleeves (not shown) can be included in the infusate holder **601.** FIG. 6A illustrates the infusate holder **601** with the lid **614** detached, FIG. 6B illustrates the infusate holder **601** after attaching the lid **614,** and FIG. 6C is a cutaway view of the infusate holder **601** after attaching the lid **614.** Upwardly extending interior walls **602, 603, 604,** and **605** define interior compartment **608** for holding a sodium bicarbonate infusate sleeve **609,** interior compartment **607** for holding a sodium chloride infusate sleeve **610,** and interior compartment **606** for holding an additional infusate sleeve or infusate container (not shown). The sodium bicarbonate infusate sleeve **609** and sodium chloride infusate sleeve **610** can be integrally formed with the lid **614.** The lid **614** performs the same functions as the rigid disks described with reference to FIG.'s 1-5. When the lid **614** is placed on the infusate holder **601,** as illustrated in FIG. 6B, the sodium bicarbonate infusate sleeve **609** and sodium chloride infusate sleeve **610** are placed within interior compartments **608** and **607** respectively. Fluid connector **611** provides for fluid ingress and egress from sodium bicarbonate infusate sleeve **609,** and fluid connector **612** provides for fluid ingress and egress from sodium chloride infusate sleeve **610.** Fluid connector **613** can connect to a disposable or non-disposable cation infusate container (not shown). Handle **615** can be included for easy maneuverability of the infusate holder **601.**

To use the disposable sodium chloride infusate sleeve **610** and sodium bicarbonate infusate sleeve **609,** fluid from a dialysate flow path (not shown) is added to solid infusate sources within the infusate sleeve. The addition of fluid from the dialysate flow path pressurizes the infusate sleeves. The interior and exterior walls of interior compartments **606, 607,** and **608** provide support for the pressurized flexible infusate sleeves, preventing the infusate sleeves from tearing during use, as illustrated in FIG. 6C. The interior compartment **608** and disposable sodium bicarbonate infusate sleeve **609** can each have a tapered bottom portion (not shown in FIG.'s 6A-C) to increase efficiency of sodium bicarbonate delivery. A tapered bottom portion of the sodium bicarbonate infusate sleeve **609** increases delivery efficiency of sodium bicarbonate from the sodium bicarbonate sleeve **609** from about 50% to over 90% as compared to a sodium bicarbonate infusate sleeve **609** without a tapered bottom portion.

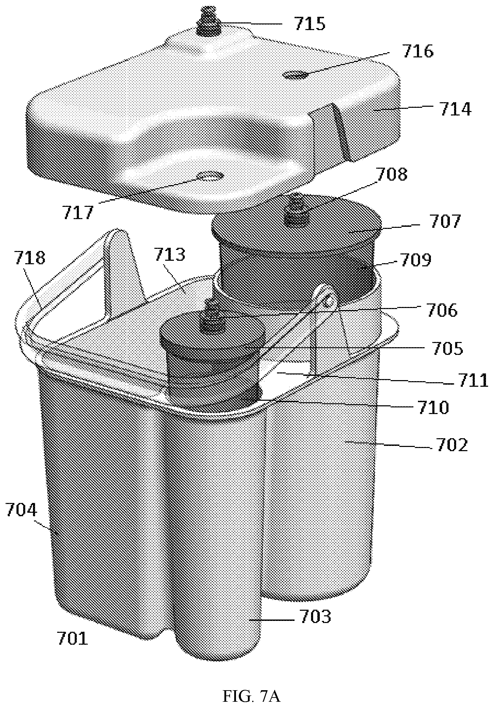

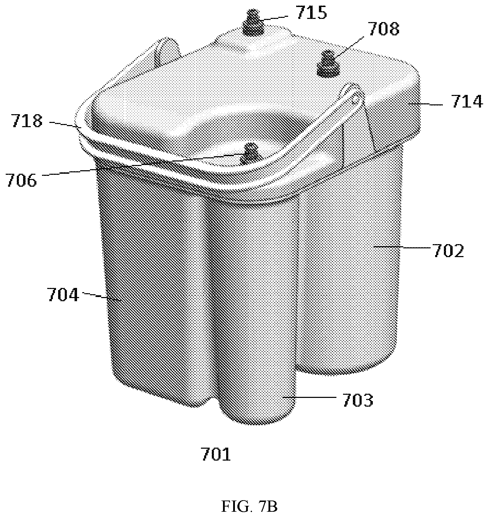

FIG.'s 7A-B illustrate an infusate holder **701** with a reusable lid **714** formed separately from disposable sodium bicarbonate infusate sleeve **709** and sodium chloride infusate sleeve **710.** FIG. 7A illustrates the infusate holder **701** with the lid **714** detached, and FIG. 7B illustrates the infusate holder **701** after attaching the lid **714.** The disposable sodium bicarbonate infusate sleeve **709** and sodium chloride infusate sleeve **710** can be integrally formed with or attached to rigid disks **707** and **705,** respectively. The rigid disks **707** and **705** can include connectors **708** and **706** for connection to a dialysis system. Exterior walls **702, 703** and **704,** as well as interior wall **711** can form an interior compartment for the sodium bicarbonate infusate sleeve **709** and sodium chloride infusate sleeve **710,** as well as one or more additional interior compartments **713** for additional infusate sleeves (not shown). The lid **714** can include opening **716** for insertion of connector **708** and opening **717** for connector **706.** The openings **716** and **717** are aligned with the interior compartments for insertion of the connectors **708** and **706.** An additional connector **715** can be included in the lid **714** for connection to a cation infusate sleeve or other infusate sleeve (not shown). Alternatively, the other infusate sleeve can include a connector, and a third opening can be included in the lid **714** in place of connector **715.** Handle **718** can be included for easy maneuverability of the infusate holder **701.**

One skilled in the art will understand that various combinations and/or modifications and variations can be made in the described systems and methods depending upon the specific needs for operation.

## Claims

1. An infusate sleeve (100), comprising:
a sleeve body (101);
a filter (104) inside of the sleeve body and sealed to the sleeve body separating the sleeve body into a top portion and a bottom portion;
a rigid disk (102) sealed to the top portion of the sleeve body; the rigid disk covering an opening in the top portion of the sleeve body;
a second opening in the bottom portion of the sleeve body;
a draw tube (105) connected to the rigid disk; the draw tube downwardly extending from the rigid disk through the filter and into the bottom portion; and
a fluid connector (103) connected to an outside of the rigid disk; the fluid connector in fluid connection with the draw tube.

2. The infusate sleeve of claim 1, wherein the filter and rigid disk are heat sealed to the sleeve body.

3. The infusate sleeve of claim 1 or claim 2, wherein the sleeve body is flexible.

4. The infusate sleeve of claim 1 of claim 2, wherein the sleeve body is rigid.

5. The infusate sleeve of any preceding claim, wherein the fluid connector is a bi-channel connector.

6. The infusate sleeve of claim 5, wherein a first channel in the bi-channel connector is fluidly connected to the draw tube.

7. The infusate sleeve of any preceding claim, further comprising a cup covering the opening in the bottom portion of the sleeve body; the cup sealed to the sleeve body.

8. The infusate sleeve of claim 7, wherein the filter is sealed to the cup.

9. The infusate sleeve of any preceding claim , wherein the filter is either a mesh or frit filter.

10. An infusate container (200), comprising:
an infusate container body (206);
the infusate sleeve (100) of any of claims 1 to 9 inside the container body; and
a cap (207); wherein the fluid connector (103) extends through an opening in the cap.

11. The infusate container of claim 10, wherein the infusate sleeve is flexible,
or wherein the infusate sleeve is rigid.

12. A dialysis system, comprising:
a dialysate flow path;
one or more fluid connectors fluidly connecting one or more infusate containers to the dialysate flow path;
wherein the one or more infusate containers comprise an infusate container body, an infusate sleeve of any of claims 1 to 9 inside the container body, and a cap; wherein the fluid connector extends through an opening in the cap; and
at least one pump connected to a fluid line fluidly connected to the fluid connectors.

13. The dialysis system of claim 12, wherein the one or more fluid connectors are bi-channel connectors.

14. The dialysis system of claim 13, wherein a first channel of the bi-channel connector fluidly connects the draw tube to a first fluid line; and wherein a second channel of the bi-channel connector fluidly connects the infusate sleeve to a second fluid line.

15. The dialysis system of any of claims 12-14, wherein the infusate sleeve contains sodium chloride, sodium bicarbonate, a cation infusate, or combinations thereof.

## Patentansprüche

1. Infusathülse (100), umfassend:
einen Hülsenkörper (101);
einen Filter (104) innerhalb des Hülsenkörpers, der an dem Hülsenkörper abgedichtet ist und den Hülsenkörper in einen oberen Abschnitt und einen unteren Abschnitt trennt;
eine starre Scheibe (102), die an dem oberen Abschnitt des Hülsenkörpers abgedichtet ist;
wobei die starre Scheibe eine Öffnung im oberen Teil des Hülsenkörpers bedeckt;
eine zweite Öffnung im unteren Teil des Hülsenkörpers;
ein Zugrohr (105), das mit der starren Scheibe verbunden ist;
wobei sich das Zugrohr von der starren Scheibe nach unten durch den Filter in den unteren Teil erstreckt; und
einen Fluidverbinder (103), der mit einer Außenseite der starren Scheibe verbunden ist;
wobei sich der Fluidverbinder in Fluidverbindung mit dem Zugrohr befindet.

2. Infusathülse nach Anspruch 1, wobei der Filter und die starre Scheibe mit dem Hülsenkörper heißversiegelt sind.

3. Infusathülse nach Anspruch 1 oder Anspruch 2, wobei der Hülsenkörper flexibel ist.

4. Infusathülse nach Anspruch 1 von Anspruch 2, wobei der Hülsenkörper starr ist.

5. Infusathülse nach einem der vorhergehenden Ansprüche, wobei der Fluidverbinder ein Zweikanalverbinder ist.

6. Infusathülse nach Anspruch 5, wobei ein erster Kanal in dem Zweikanalverbinder fluidisch mit dem Zugrohr verbunden ist.

7. Infusathülse nach einem der vorhergehenden Ansprüche, ferner umfassend einen Napf, der die Öffnung im unteren Abschnitt des Hülsenkörpers bedeckt;
wobei der Napf am Hülsenkörper abgedichtet ist.

8. Infusathülse nach Anspruch 7, wobei der Filter am Napf abgedichtet ist.

9. Infusathülse nach einem der vorhergehenden Ansprüche, wobei der Filter entweder ein Sieb- oder ein Frittenfilter ist.

10. Infusatbehälter (200), umfassend:
einen Infusatbehälter (206), die Infusathülse (100) nach einem der Ansprüche 1 bis 9 innerhalb des Behälterkörpers; und
eine Kappe (207);
wobei sich der Fluidverbinder (103) durch eine Öffnung in der Kappe erstreckt.

11. Infusatbehälter nach Anspruch 10, wobei die Infusathülse flexibel ist oder wobei die Infusathülse starr ist.

12. Dialysesystem, umfassend:
einen Dialysatströmungsweg;
einen oder mehrere Fluidverbinder, die einen oder mehrere Infusatbehälter fluidisch mit dem Dialysatströmungsweg verbinden;
wobei der eine oder die mehreren Infusatbehälter einen Infusatbehälterkörper, eine Infusathülse nach einem der Ansprüche 1 bis 9 innerhalb des Behälterkörpers und eine Kappe umfassen;
wobei sich der Fluidverbinder durch eine Öffnung in der Kappe erstreckt; und
mindestens eine Pumpe fluidisch mit einer Fluidleitung verbunden ist, die mit den Fluidverbindern verbunden ist.

13. Dialysesystem nach Anspruch 12, wobei der eine oder die mehreren Fluidverbinder Zweikanalverbinder sind.

14. Dialysesystem nach Anspruch 13, wobei ein erster Kanal des Zweikanalverbinders das Zugrohr fluidisch mit einer ersten Fluidleitung verbindet; und
wobei ein zweiter Kanal des Zweikanalverbinders die Infusathülse fluidisch mit einer zweiten Fluidleitung verbindet.

15. Dialysesystem nach einem der Ansprüche 12 bis 14, wobei die Infusathülse Natriumchlorid, Natriumbicarbonat, ein Kationeninfusat oder Kombinationen davon enthält.

## Revendications

1. Manchon de solution intraveineuse (100), comprenant :
un corps de manchon (101) ;
un filtre (104)
à l'intérieur du corps de manchon et scellé au corps de manchon séparant le corps de manchon en une partie supérieure et une partie inférieure ;
un disque rigide (102)
scellé à la partie supérieure du corps de manchon ; le disque rigide recouvrant une ouverture dans la partie supérieure du corps de manchon ;
une seconde ouverture dans la partie inférieure du corps de manchon ;
un tube à jet (105)
raccordé au disque rigide ; le tube à jet s'étendant vers le bas depuis le disque rigide à travers le filtre et dans la partie inférieure ; et
un raccord de fluide (103)
raccordé à un côté extérieur du disque rigide ; le raccord de fluide en raccord fluidique avec le tube à jet.

2. Manchon de solution intraveineuse selon la revendication 1, le filtre et le disque rigide étant thermosoudés au corps de manchon.

3. Manchon de solution intraveineuse selon la revendication 1 ou la revendication 2, le corps de manchon étant flexible.

4. Manchon de solution intraveineuse selon la revendication 1 ou la revendication 2, le corps de manchon étant rigide.

5. Manchon de solution intraveineuse selon l'une quelconque des revendications précédentes, le raccord de fluide étant un raccord à deux canaux.

6. Manchon de solution intraveineuse selon la revendication 5, un premier canal dans le raccord à deux canaux étant raccordé fluidiquement au tube à jet.

7. Manchon de solution intraveineuse selon l'une quelconque des revendications précédentes, comprenant en outre une coupelle recouvrant l'ouverture dans la partie inférieure du corps de manchon ; la coupelle étant scellée au corps de manchon.

8. Manchon de solution intraveineuse selon la revendication 7, le filtre étant scellé à la coupelle.

9. Manchon de solution intraveineuse selon l'une quelconque des revendications précédentes, le filtre étant soit un filtre à mailles, soit un filtre fritté.

10. Récipient de solution intraveineuse (200), comprenant :
un corps de récipient de solution intraveineuse (206),
le manchon de solution intraveineuse (100) selon l'une quelconque des revendications 1 à 9 à l'intérieur du corps de récipient ; et
un couvercle (207) ; le raccord de fluide (103) s'étendant à travers une ouverture dans le couvercle.

11. Récipient de solution intraveineuse selon la revendication 10, le manchon de solution intraveineuse étant flexible, ou le manchon de solution intraveineuse étant rigide.

12. Système de dialyse, comprenant :
un trajet d'écoulement de dialysat ;
un ou plusieurs raccords de fluide raccordant fluidiquement un ou plusieurs récipients de solution intraveineuse au trajet d'écoulement de dialysat ;
le ou les récipients de solution intraveineuse comprenant un corps de récipient de solution intraveineuse, un manchon de solution intraveineuse selon l'une quelconque des revendications 1 à 9 à l'intérieur du corps de récipient, et un couvercle ; le raccord de fluide s'étendant à travers une ouverture dans le couvercle ; et
au moins une pompe raccordée à une trajectoire de fluide raccordée fluidiquement aux raccords de fluide.

13. Système de dialyse selon la revendication 12, le ou les raccords de fluide étant des raccords à deux canaux.

14. Système de dialyse selon la revendication 13, un premier canal du raccord à deux canaux raccordant fluidiquement le tube à jet à une première trajectoire de fluide ; et un second canal du raccord à deux canaux raccordant fluidiquement le manchon de solution intraveineuse à une seconde trajectoire de fluide.

15. Système de dialyse selon l'une quelconque des revendications 12 à 14, le manchon de solution intraveineuse contenant du chlorure de sodium, du bicarbonate de sodium, une solution intraveineuse de cations ou leurs combinaisons.
